# EUROPEAN PATENT SPECIFICATION

(11) **EP 2 133 682 B1**
(45) Date of publication and mention of the grant of the patent: **24.10.2012**
(21) Application number: 09162357.9
(22) Date of filing: 10.06.2009
(51) Int. Cl.: G01N 1/24, G01N 1/22, G01N 15/06

(54) **Method and equipment for measuring dust formation**
Verfahren und Vorrichtung zur Messung des Staubgehaltes
Procédé et dispositif pour la mesure du contenu en poussière

(30) Priority: 13.06.2008 FI 20085588
(43) Date of publication of application: 16.12.2009
(73) Proprietor: Lilja, Jarmo, 33800 Tampere (FI); Arvela, Pasi, 33300 Tampere (FI); Pitkänen, Juhani, 33710 Tampere (FI); Nikkilä, Arto, 33560 Tampere (FI)
(72) Inventor: Lilja, Jarmo, 33800 Tampere (FI); Arvela, Pasi, 33300 Tampere (FI); Pitkänen, Juhani, 33710 Tampere (FI); Nikkilä, Arto, 33560 Tampere (FI)
(74) Representative: Kaukonen, Juha Veikko

(56) References cited:
- EP-A1- 1 413 875
- WO-A1-97/14033
- JP-A- 9 039 204
- US-B1- 6 573 836
- MONTZ K W; BEDDOW J K; BUTLER P B: "ADHESION AND REMOVAL OF PARTICULATE CONTAMINANTS IN A HIGH-DECIBEL ACOUSTIC FIELD." POWDER TECHNOLOGY, vol. 55, no. 2, 1 June 1988 (1988-06-01), pages 133-140, XP002598599

## Description

### BACKGROUND OF THE INVENTION

The invention relates to a method for measuring dust formation, the method comprising detaching dust from a product to be measured and conveying said dust to a measuring device measuring the amount of dust, bringing the product to be measured to the vicinity of a collection chamber, the collection chamber comprising an opening; detaching dust from the product to be measured by bringing the product to the condition of vibration by subjecting it to pressure waves of a gaseous medium, generated by an acoustic vibrator; and conveying dust via said opening into the collection chamber and further to the measuring device.

Further, the invention relates to equipment for measuring dust formation, the equipment comprising means for detaching dust from a product to be measured, a collection chamber arranged to receive dust detached from the product to be measured, and a measuring device measuring the amount of dust and being connected to the collection chamber the means for detaching dust from the product to be measured comprises an acoustic vibrator arranged to generate wave motion in gaseous medium; that the collection chamber comprises an opening arranged opposite to the acoustic vibrator; and that there is a space arranged between the opening of the collection chamber and the acoustic vibrator to receive the product to be measured, and the equipment further comprises a replacement channel for conveying replacement gaseous medium into the collection chamber to replace the amount of gaseous medium conveyed to the measuring device.

In manufacturing and treating paper, cardboard, other corresponding fibre-based material and some other materials, dust formation occurs. Dust is detached from the material to be made or treated. For instance, dust being detached from paper may comprise organic fibres, paper coating and filler. Dust may cause problems when gathering on surfaces of a printing machine, for example.

Above-mentioned dust is, as known, measured with methods and devices in which the product to be measured is arranged in a closable sample box where different means are used for the product to detach dust. For example, Finnish patent FI 110 213 B uses compressed-air blowing, whereas in Japanese publication JP 60 058 527 a product is attached to the end of an arm, the arm being then vibrated. However, said methods and devices have the problem that their use is complex and slow. Another problem relating to the known solutions is that they cannot be used for real-time measurements during the manufacturing or other treatment of the product. US 6,573,836 discloses features of preamble of the independent claims. In this document the entrance of ambient dust in the collection chamber is prevented by a container that is closed by a cover sealed against the container.

### BRIEF DESCRIPTION OF THE INVENTION

An object of this invention is to provide a novel and improved method and equipment for measuring dust formation

The method according to the invention is characterized by surrounding the space between the edges of the opening of the collection chamber and the product to be measured by means of a dynamic insulator which is formed by conveying gaseous medium to said space with a flow greater than a flow of gaseous medium to the measuring device.

The device according to the invention is **characterized in that** the replacement channel comprises a blow opening leading to the edges of the opening of the collection chamber or to the vicinity thereof, the blow opening being arranged to convey gas to the space between the edges of the opening of the collection chamber and the product to be measured with a flow greater than the flow of gas to the measuring device.

An idea of the invention is that the product to be measured is not taken into the inside of the collection chamber but that the collection chamber comprises an opening to the vicinity of which the product is taken, and that the product is caused to vibrate by subjecting it to pressure waves generated with an acoustic vibrator.

An advantage of the invention is that the dust formation measurement can be performed quickly and, if required, on-line from a moving product.

The idea of the invention is that the space between the edges of the opening of the collection chamber and the product to be measured is surrounded by a dynamic insulator which is formed by conveying gas to this space at a pressure higher than the gas pressure in the surroundings of the collection chamber. An advantage is that the dust in the surroundings cannot affect the measurement results.

### BRIEF DESCRIPTION OF THE FIGURES

Some embodiments of the invention are described in greater detail in the attached drawings, in which
Figure 1 shows schematically a side view of equipment according to the invention;
Figure 2 shows schematically a side view of second equipment according to the invention; and
Figure 3 shows schematically a side view of a detail of the equipment according to the invention.

For the sake of clarity, embodiments of the invention are shown simplified in the figures. Similar parts are denoted with the same reference numerals in the figures.

### DETAILED DESCRIPTION OF THE INVENTION

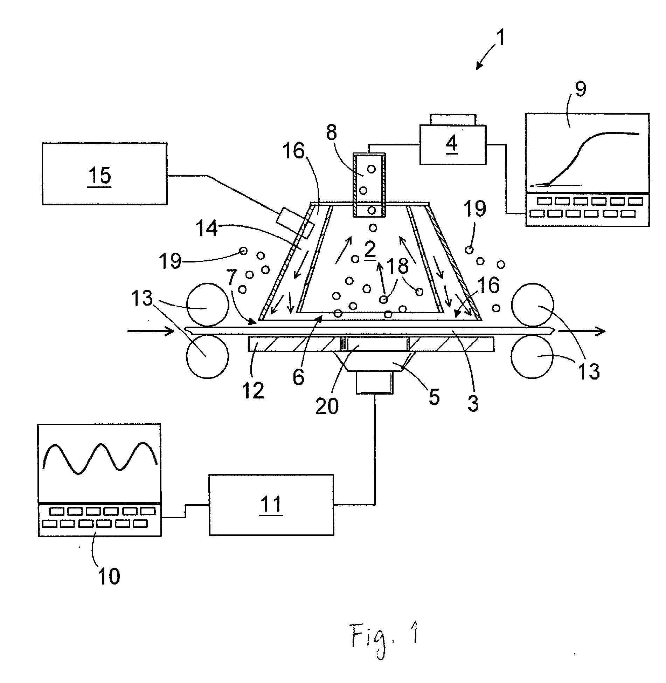

Figure 1 shows schematically a side view of equipment according to the invention. Equipment 1 comprises a collection chamber 2 arranged to receive dust detached from a product 3 to be measured.

The collection chamber 2 is manufactured of metal or plastic, for example, preferably of semiconducting plastic, such as plastic used in ESD (Electrostatic Discharge) applications. Electrically conductive or semiconducting material reduces electric adhesion of particles to the walls of the collection chamber 2. The lower part of the collection chamber 2 is provided with an opening 6. The opening 6 is, in this case of the size of the whole side of the chamber but its size may naturally be different.

The product 3 is arranged in the vicinity of the opening 6, in this case at a short distance from the opening 6. The product 3 may be of paper or cardboard, for example, or another corresponding relatively thin product.

The collection chamber 2 is connected via a flow channel 8 to a measuring device 4 measuring the amount of dust. The measuring device 4 is what is called a fine particle measuring device, which is known as such, so its structure or operation is not explained in more detail in this description. It is to be noted that the measuring device 4 may also be arranged directly in the collection chamber 2.

The measurement results of the measuring device 4 are fed to a monitoring device 9, by means of which the measurement results can be monitored and processed.

The equipment 1 comprises an acoustic vibrator 5 arranged to generate wave motion, which, in turn, generates dust-detaching vibration in the product 1. The medium of the wave motion is a gaseous medium, in this case air. The medium may naturally be another gaseous substance, for example nitrogen gas, dry air or the like gas used as a protective gas.

The acoustic vibrator 5 is typically an electromechanical loudspeaker unit converting electric signal into sound. An electric signal is generated in a microprocessor-controlled signal generator 10 and amplified in an amplifier 11. The frequency range of the acoustic vibrator is typically 20 to 20 000 Hz.

The loudspeaker unit, signal generator and amplifier are devices and components known as such, so they are not described in greater detail here. Acoustic vibration may also be generated with other methods and arrangements. It is still to be noted that the equipment 1 may have more than one acoustic vibrator 5.

The signal generator 10 preferably comprises means for optimizing properties of the wave motion, such as the frequency and the amplitude, according to the properties of the product to be measured. Further, it is obvious that the energy of the wave motion is preferably controllable.

The acoustic vibrator 5 is arranged on a plane 12 functioning as a support for the product 3 in such a way that it is opposite to the opening 6 of the collection chamber. More precisely, the acoustic vibrator is, in the present embodiment, arranged at an opening 20 penetrating the plane 12. Between the opening 6 of the collection chamber and the acoustic vibrator, there is a space 7 for the product 3 to be measured. The product 3 to be measured is arranged on the plane 12 in this space 7 in such a way that the pressure waves generated by the acoustic vibrator 5 hit the product 3. In the embodiment shown in Figure 1, the pressure waves meet the surface of the product 3 on average perpendicularly, in other words the vibrator 5 is directed perpendicularly to the surface of the product 3, but it may naturally be arranged at another angle relative to the product 3.

The equipment 1 shown in Figure 1 measures dust formation of a web-like product. The equipment is provided with conveying means for conveying the product 3 relative to the collection chamber 2 between the opening 6 of the collection chamber and the acoustic vibrator 5. Said conveying means comprise rolls 13 between which the product 3 is arranged and which rolls 13 are connected to a power unit rotating the rolls 13 and conveying the product to the equipment 1 and away from it. Movement of the product 3 through the equipment 1 needs not be stopped for the duration of the measurement but the equipment 1 measures on-line the product 3 moving relative to the collection chamber.

It is naturally feasible to stop the product for the duration of the measurement if there is reason to do so. In this case, the movement 13 of the rolls is stopped or alternatively at least the rolls 13 connected to the power unit are detached from the product 3 for the duration of the measurement.

The conveying means are not necessary parts of the equipment 1. The equipment may be implemented without conveying means, and if it desirable to measure the product 3 in movement, conveying means being part of the production means or treating means of the product may be utilized.

In connection with the collection chamber 2, a replacement channel 14 is arranged via which replacement air is conveyed into the collection chamber 2 to replace the amount of air that is sucked out into the collection chamber 2 and conveyed to the measuring device 4 measuring the amount of dust. Replacement air is pure air generated in a pure air device 15.

Replacement air is blown out of the replacement channel 14 via one or more blow openings 16. The replacement channel 14 forms, in this case, a kind of housing around the collection chamber 2 in such a way that the blow opening 16 of the replacement channel surrounds the opening 6 of the collection chamber substantially on all sides. Part of the air blown via the blow opening 16 flows to the surroundings from between the opening and the product 3. The flow of the air blown must be greater than the flow to the flow channel 8 and the measuring device 4. This outflow thus forms a dynamic insulator which prevents dust in the surroundings from getting into the collection chamber 2 and to the measuring device 4. The opening between the blow opening 16 and the product 3 is smaller than the opening between the opening 6 of the collection chamber and the product 3, which aspect contributes to reducing the risk of dust in the surroundings getting into the collection chamber 2.

In general terms, the measurement takes place in such a way that when on the plane 12, the product 3 to be measured is subjected to pressure waves generated by the acoustic vibrator 5. The pressure waves generate in the product 3 to be measured vibrations which detach dust from the product 3. The vibrating part of the product 3 is shown wave-like in the figure. The waveform of the pressure waves may also be different; it may, for instance, vary during the measurement or it may be superposition of several waveforms.

Dust detached from the product 3 to be measured is absorbed via the collection chamber 2 to the measuring device 4, which measures the amount of dust. The dust 18 arriving in the measuring device 4 originates substantially completely or even completely from the product 3 because pure air blowing of the replacement channel prevents dust 19 in the surroundings from getting into the collection chamber 2 and to the measuring device 4. Owing to this, the measurement result is at least not essentially dependent on the dust level of the surroundings.

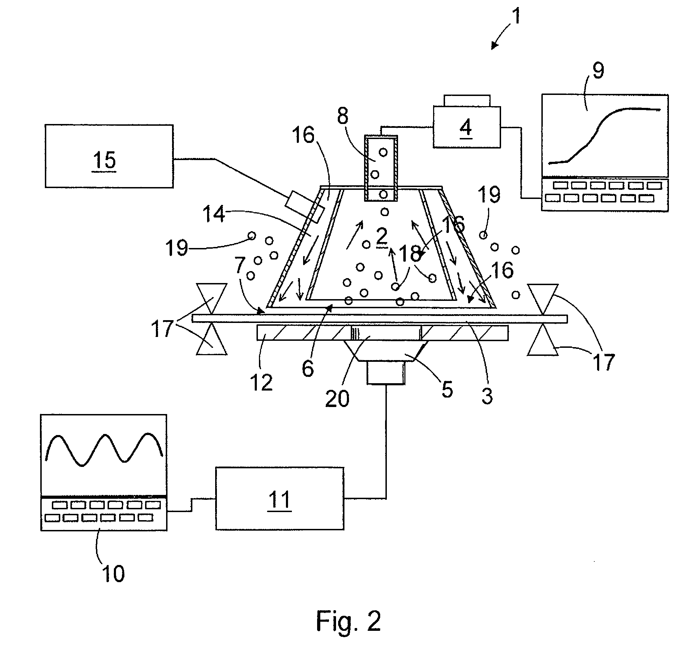

Figure 2 shows schematically a side view of second equipment according to the invention. The equipment 1 of Figure 2 deviates from the equipment shown in Figure 1 in that the equipment 1 now comprises attaching means 17 with which the product 3 to be measured is detachably attached to the apparatus 1 for the duration of the measurement. The attaching means 17 keep the product 3 in place between the opening 6 of the collection chamber and the acoustic vibrator 5. The attaching means 17 may be known clamps or the like means with which the product 3 is preferably easily attachable and detachable.

In some embodiments of the equipment, the collection chamber 2 may be moved away from top of the plane 12, or the plane 12 may be moved away from under the collection chamber 2 in such a way that it is easy to attach the product 3 to the apparatus 1.

The equipment 1 shown in Figure 2 is particularly suitable for measuring dust formation of sheet-like products.

The equipment 1 according to the invention may be arranged in a manufacturing apparatus of a web-like product, where it measures the product 3 moving relative to the collection chamber 2 in real time. The manufacturing apparatus may be, for example, a paper or cardboard machine making paper or cardboard.

The equipment may also be arranged in treatment equipment of a web-like product for real-time measurement. Such treatment equipment may be, for instance, a printing line or an analyzer device for a web-like product.

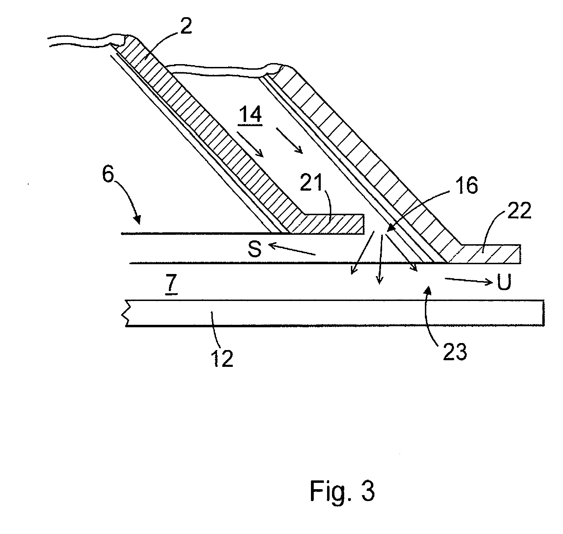

Figure 3 shows schematically a side and cut-out view of a detail of the equipment according to the invention. It can be seen that the opening 6 of the collection chamber is at a higher level than the blow opening 16. At the lower edge of the collection chamber 2, around the opening 6, a substantially protruding collar 21 has been formed which guides the air flow from the replacement channel 14 and increases its flow rate. Air flows both inwards S and outwards U relative to the collection chamber 2. The air flowing inwards S replaces air flowing from the measuring chamber in the direction of the measuring device 4. The air flowing outwards U forms a dynamic insulator 23 which prevents air in the surroundings from mixing into the air flow to be conveyed to the measuring device 4.

On the outer casing of the replacement channel 14, around the blow opening 16, a second protruding collar 22 has been formed which guides the air flowing outwards U.

It is to be noted that the equipment according to the invention can naturally be implemented in another way: for example the first and/or the second collar 21, 22 may be left out. The replacement channel 14 does not necessarily have to be a continuous structure surrounding the collection chamber 2 but it may be a pipe or hose or another channel connected to the collection chamber 2, for instance directly through the wall of the collection chamber 2. Instead of one blow opening 16, a plurality of blow openings 16 may be used which are arranged, for example, at suitable spacings or side by side close to each other around the opening 6 of the collection chamber.

Features of some embodiments of the invention are still repeated here:

The gaseous medium may be air.

The product to be measured, moving relative to the collection chamber, may be measured in real time.

A sheet-like product to be measured may be measured.

The equipment may be fitted in a manufacturing apparatus of a web-like product and arranged to measure the product to be measured, which is moving relative to the collection chamber, in real time.

The frequency generated by an acoustic vibrator may be selected from a frequency range of 20 to 20 000 Hz, for example.

The means for detaching dust from the product to be measured may comprise a microprocessor-controlled signal generator which may comprise means for changing properties of the wave motion, such as the frequency, waveform or amplitude.

The equipment may comprise conveying means for conveying the product to be measured relative to the collection chamber between the opening of the collection chamber and the acoustic vibrator.

The equipment may comprise attaching means for keeping the product in place between the opening of the collection chamber and the acoustic vibrator.

The equipment may be arranged in a paper or cardboard machine making paper or cardboard.

The equipment may be fitted in treatment equipment of a web-like product, such as a printing machine or an analyzer device, and arranged to measure the product to be measured, which is moving relative to the collection chamber, in real time.

In some cases, features presented in this application may be used as such, irrespective of other features. On the other hand, features presented in this application may be, if required, combined to form various combinations.

The drawings and the related description are only intended to illustrate the idea of the invention. Details of the invention may vary within the claims.

## Claims

1. A method for measuring dust formation, the method comprising
detaching dust from a product (3) to be measured and
conveying said dust to a measuring device (4) measuring the amount of dust,
bringing the product (3) to be measured to the vicinity of a collection chamber (2), the collection chamber comprising an opening (6);
detaching dust from the product (3) to be measured by bringing the product to the condition of vibration by subjecting it to pressure waves of a gaseous medium, generated by an acoustic vibrator (5); and
conveying dust via said opening (6) by sucking gaseous medium into the collection chamber (2) and further to the measuring device (4) **characterized by**
surrounding the space between the edges of the opening (6) of the collection chamber and the product (3) to be measured by means of a dynamic insulator which is formed by conveying gaseous medium to said space with a flow greater than a flow of gaseous medium to the measuring device (4).

2. A method according to claim 1, **characterized by** the product (3) to be measured being a web-like product (3), the product being in movement relative to the collection chamber (2):

3. A method according to claim 1, **characterized by** measuring the product (3) to be measured, the product being immovable relative to the collection chamber (2).

4. A method according to any one of the preceding claims, **characterized by** subjecting the pressure waves substantially perpendicularly to the surface of the product (3) to be measured.

5. A method according to any one of the preceding claims, **characterized by** the acoustic vibrator (5) being a loudspeaker.

6. A method according to any one of the preceding claims, **characterized by** using the acoustic vibrator (5) for generating pressure waves the frequency of which is in a frequency range of 20 to 20 000 Hz.

7. A method according to any one of the preceding claims, **characterized by** measuring dust formation of paper or another fibre-based product.

8. Equipment for measuring dust formation, the equipment (1) comprising
means for detaching dust from a product (3) to be measured,
a collection chamber (2) arranged to receive dust detached from the product (3) to be measured, and
a measuring device (4) measuring the amount of dust and being connected to the collection chamber (2),
the means for detaching dust from the product (3) to be measured comprises an acoustic vibrator (5) arranged to generate wave motion in gaseous medium;
means for sucking gaseous medium into the collection chamber (2), the collection chamber (2) comprises an opening (6) arranged opposite to the acoustic vibrator (5); and
there is a space (7) arranged between the opening (6) of the collection chamber and the acoustic vibrator (5) to receive the product (3) to be measured, and
the equipment further comprises a replacement channel (14) for conveying replacement gaseous medium into the collection chamber (2) to replace the amount of gaseous medium conveyed to the measuring device (4), **characterized in that**
the replacement channel (14) comprises a blow opening (16) leading to the edges of the opening (6) of the collection chamber or to the vicinity thereof, the blow opening being arranged to convey gaseous medium to the space between the edges of the opening (6) of the collection chamber and the product (3) to be measured with a flow greater than the flow of gaseous medium to the measuring device (4).

9. Equipment according to claim8, **characterized in that** the acoustic vibrator (5) is a loudspeaker and that the gaseous medium is air.

10. Equipment according to any one of claims 8 to 9, **characterized in that** the acoustic vibrator (5) is directed in such a way that it is perpendicular to the surface of the product (3) to be measured.

11. Equipment according to any one of claims 8 to 10, **characterized in that** the blow opening (16) is positioned closer to the product (3) to be measured than the opening (6) of the collection chamber.

## Patentansprüche

1. Verfahren zur Messung der Staubbildung, wobei das Verfahren folgendes umfasst
das Loslösen von Staub von einem zu messenden Produkt (3) und
das Befördern des Staubs zu einer Messvorrichtung (4), die die Staubmenge misst;
das Bringen des zu messenden Produkts (3) in die Nähe einer Auffangkammer (2), wobei die Auffangkammer eine Öffnung (6) umfasst,
das Loslösen des Staubs von dem zu messenden Produkt (3) durch Bringen des Produkts in den Zustand des Vibrierens durch Unterwerfen desselben Druckwellen eines gasförmigen Mediums, die durch einen akustischen Vibrator (5) erzeugt werden; und
das Befördern des Staubs durch die Öffnung (6) durch Saugen von gasförmigem Medium in die Auffangkammer (2) und weiter zu der Messvorrichtung (4),
**gekennzeichnet durch**
Umgeben des Raums zwischen den Kanten der Öffnung (6) der Auffangkammer und dem zu messenden Produkt (3) durch einen dynamischen Isolator, der **durch** Befördern von gasförmigem Medium zu dem Raum mit einer Strömung gebildet wird, die höher ist als die Strömung von gasförmigem Medium zu der Messvorrichtung (4).

2. Verfahren nach Anspruch 1, **dadurch gekennzeichnet, dass** das zu messende Produkt (3) ein bahnartiges Produkt (3) ist, wobei das Produkt sich mit Bezug auf die Auffangkammer (2) in Bewegung befindet.

3. Verfahren nach Anspruch 1, **gekennzeichnet durch** Messen des zu messenden Produkts (3), wobei das Produkt mit Bezug auf die Auffangkammer (2) unbeweglich ist.

4. Verfahren nach einem der vorhergehenden Ansprüche, **gekennzeichnet durch** Unterwerfen der Druckwellen im Wesentlichen senkrecht zu der Oberfläche des zu messenden Produkts (3).

5. Verfahren nach einem der vorhergehenden Ansprüche, **dadurch gekennzeichnet, dass** der akustische Vibrator (5) ein Lautsprecher ist.

6. Verfahren nach einem der vorhergehenden Ansprüche, **gekennzeichnet durch** Anwenden des akustischen Vibrators (5) zum Erzeugen von Druckwellen, deren Frequenz eine Frequenz im Bereich von 20 bis 20.000 Hz ist.

7. Verfahren nach einem der vorhergehenden Ansprüche, **gekennzeichnet durch** Messen der Staubbildung von Papier oder einem anderen Produkt auf Faserbasis.

8. Vorrichtung zum Messen von Staubbildung, wobei die Vorrichtung (1) Folgendes umfasst
Vorrichtung zum Loslösen von Staub von einem zu messenden Produkt (3),
eine Auffangkammer (2), die zum Aufnehmen von Staub, der von dem zu messenden Produkt losgelöst wird, angeordnet ist und
eine Messvorrichtung (4), die die Staubmenge misst und an die Auffangkammer (2) angeschlossen ist;
wobei die Vorrichtung zum Loslösen von Staub von dem zu messenden Produkt (3) einen akustischen Vibrator (5) umfasst, der zum Erzeugen von Wellenbewegung in dem gasförmigen Medium angeordnet ist;
Vorrichtung zum Saugen von gasförmigem Medium in die Auffangkammer (2),
wobei die Auffangkammer (2) eine Öffnung (6) umfasst, die dem akustischen Vibrator (5) gegenüber angeordnet ist; und
ein Raum (7) zwischen der Öffnung (6) der Auffangkammer und dem akustischen Vibrator (5) angeordnet ist, um das zu messende Produkt (3) aufzunehmen; und
die Vorrichtung des Weiteren einen Ersatzkanal (14) zum Befördern des gasförmigen Ersatzmediums in die Auffangkammer (2) umfasst, um die Menge an gasförmigem Medium, die zu der Messvorrichtung (4) befördert wird, zu ersetzen,
**dadurch gekennzeichnet, dass**
der Ersatzkanal (14) eine Blasöffnung (16) umfasst, die zu den Kanten der Öffnung (6) der Auffangkammer oder in die Nähe davon führt, wobei die Blasöffnung so angeordnet ist, dass sie gasförmiges Medium zu dem Raum zwischen den Kanten der Öffnung (6) der Auffangkammer und dem zu messenden Produkt (3) mit einer Strömung transportiert, die höher ist als die Strömung des gasförmigen Mediums zu der Messvorrichtung (4).

9. Vorrichtung nach Anspruch 8, **dadurch gekennzeichnet, dass**
der akustische Vibrator (5) ein Lautsprecher ist und dass das gasförmige Medium Luft ist.

10. Vorrichtung nach einem der Ansprüche 8 bis 9, **dadurch gekennzeichnet, dass** der akustische Vibrator (5) derart ausgerichtet wird, dass er senkrecht zu der Oberfläche des zu messenden Produkts (3) liegt.

11. Vorrichtung nach einem der Ansprüche 8 bis 10, **dadurch gekennzeichnet, dass** die Blasöffnung (16) näher zu der zu messenden Produkt (3) als der Öffnung (6) der Auffangkammer positioniert ist.

## Revendications

1. Un procédé pour la mesure de la formation de poussière, le procédé comprenant
le fait de détacher de la poussière d'un produit (3) à mesurer, et
le fait de transporter ladite poussière vers un dispositif de mesure (4) apte à mesurer la quantité de poussière,
le fait d'amener le produit (3) à mesurer à proximité d'une chambre de collecte (2), la chambre de collecte comprenant une ouverture (6) ;
le fait de détacher la poussière du produit (3) à mesurer en mettant l'article sous une condition de vibration en le soumettant à des ondes de pression d'un milieu gazeux, générées par un vibreur acoustique (5) ; et
le fait de transporter la poussière par l'intermédiaire de ladite ouverture (6) en aspirant le fluide gazeux dans la chambre de collecte (2) et ensuite dans le dispositif de mesure (4), **caractérisé par**
le fait d'entourer l'espace situé entre les bords de l'ouverture (6) de la chambre de collecte et le produit (3) à mesurer au moyen d'un isolant dynamique qui est formé en amenant le fluide gazeux dans ledit espace avec un débit supérieur à un débit du fluide gazeux dans l'appareil de mesure (4).

2. Un procédé selon la revendication 1, **caractérisé en ce que** le produit (3) à mesurer est un produit en forme de bande (3), le produit étant en mouvement par rapport à la chambre de collecte (2).

3. Un procédé selon la revendication 1, **caractérisé par** le fait de mesurer le produit (3) à mesurer, le produit étant immobile par rapport à la chambre de collecte (2).

4. Un procédé selon l'une quelconque des revendications précédentes, **caractérisé par** le fait de générer les ondes de pression sensiblement perpendiculairement à la surface du produit (3) à mesurer.

5. Un procédé selon l'une quelconque des revendications précédentes, **caractérisé par le fait que** le vibreur acoustique (5) est un haut-parleur.

6. Un procédé selon l'une quelconque des revendications précédentes, **caractérisé par** l'utilisation du vibreur acoustique (5) pour générer des ondes de pression dont la fréquence est dans une plage de fréquence allant de 20 à 20 000 Hz.

7. Un procédé selon l'une quelconque des revendications précédentes, **caractérisé par** le fait de mesurer la formation de poussière par du papier ou un autre produit à base de fibres.

8. Appareil pour la formation de poussière de mesure, l'appareil (1) comprenant
des moyens pour détacher de la poussière à partir d'un produit (3) à mesurer,
une chambre de collecte (2) agencée pour recevoir la poussière détachée de produit (3) à mesurer, et
un dispositif de mesure (4) mesurant de la quantité de poussière et étant relié à la chambre de collecte (2) ;
les moyens pour détacher la poussière à partir du produit (3) à mesurer comprennent un vibreur acoustique (5) agencé pour générer un mouvement d'onde dans un fluide gazeux ;
des moyens pour aspirer le fluide gazeux dans la chambre de collecte (2) ;
la chambre de collecte (2) comprend une ouverture (6) aménagée à l'opposé du vibreur acoustique (5), et
il existe un espace (7) agencé entre l'ouverture (6) de la chambre de collecte et le vibreur acoustique (5) pour recevoir le produit (3) à mesurer, et
l'appareil comprenant en outre un canal de remplacement (14) pour transporter un fluide gazeux de remplacement dans la chambre de collecte (2) pour remplacer la quantité de fluide gazeux transmis à l'appareil de mesure (4), **caractérisé en ce que**
le canal de remplacement (14) comprend une ouverture de soufflage (16) conduisant aux bords de l'ouverture (6) de la chambre de collecte ou à proximité de ceux-ci, l'ouverture de soufflage étant agencée pour transporter du fluide gazeux jusqu'à l'espace situé entre les bords de l'ouverture (6) de la chambre de collecte et le produit (3) à mesurer avec un débit supérieur au débit de fluide gazeux dans l'appareil de mesure (4).

9. Appareil selon la revendication 8, **caractérisé en ce que** le vibreur acoustique (5) est un haut-parleur et **en ce que** le fluide gazeux est de l'air.

10. Appareil selon l'une quelconque des revendications 8 à 9, **caractérisé en ce que** le vibreur acoustique (5) est dirigé de telle sorte qu'il soit perpendiculaire à la surface du produit (3) à mesurer.

11. Appareil selon l'une quelconque des revendications 8 à 10, **caractérisé en ce que** l'ouverture de soufiflage (16) est positionnée plus près du produit (3) à mesurer que l'ouverture (6) de la chambre de collecte.
